# EUROPEAN PATENT APPLICATION

(11) **EP 2 143 823 A2**
(43) Date of publication of application: **13.01.2010**
(21) Application number: 09251677.2
(22) Date of filing: 29.06.2009
(51) Int. Cl.: C23F 1/26, A61F 2/30, A61L 27/06, A61L 27/56, B22F 7/00

(54) **Open-celled metal implants with roughened surfaces**

(30) Priority: 30.06.2008 US 76861 P
(71) Applicant: DePuy Products, Inc., Warsaw, IN 46581 (US)
(72) Inventor: Liu, Hengda Derek, Warsaw, IN 46580 (US); Yang, Sophie Ziaofan, Warsaw, IN 46580 (US)
(74) Representative: Belcher, Simon James

(57) **Abstract**

Processes for etching a porous titanium foam or porous titanium alloy foam involve immersing a clean, dry foam product in an aqueous acid solution comprising about 0.5 to about 5 volume percent HF and about 5 to about 20 volume percent HNO₃ for a time sufficient to achieve a desired surface roughness and heating the etched foam to remove residual titanates. The etching process increases the porosity at the surface of the foam but the etchant does not penetrate fully into the interior of the foam so that adequate mechanical properties are maintained. The etching process also increases the coefficient of friction at the foam surface. The foam may comprise an open-celled orthopaedic or dental implant, or may comprise a coating on the surface of a substrate.

## Description

The present invention relates to open-celled metal medical implants with roughened surfaces and methods of roughening the surfaces of open-celled metal implants. More particularly, the present invention relates to such implants and methods wherein the open-celled metal implant contains titanium (either pure or in an alloy form), and more particularly, sintered titanium powder, and wherein the implant is intended for use as an orthopaedic implant.

Orthopaedic implants have been used in joint replacement, such as in total knee arthroplasty, total hip arthroplasty and shoulder arthroplasty, in spinal surgery and in trauma care. There are a number of design criteria which have long been sought for orthopaedic implants including that (1) the implant should have a long useful life without losing function or initiating any adverse process response; (2) the implant should restore the normal function of the bone in which it is implanted; and (3) the implant should be capable of production on a commercial scale. In attempting to satisfy these criteria, not only should the implant support the imposed load, often of a fluctuating nature, but the interface between the implant and the bone should also withstand the load requirement.

Generally, there have been two approaches to achieving fixation at the implant/bone interface: cementing the implant into place and fixing the implant into the bone without cement. For cemented applications, a plastic cement such as polymethyl methacrylate is often used to affix an implant to bone as well as to improve the fit between the implant and the bone. For uncemented applications, short term fixation is achieved through a friction fit, and long term fixation is achieved through the ingrowth of bone into the implant; to achieve bone ingrowth, implants have been provided with irregular surfaces which mate with the bone and invite bone ingrowth such that, after a period of time, the prosthesis becomes integrated into the bone structure.

Several techniques have been used to create implants with irregular surfaces. Frequently, the implants are provided with a coating of metal elements, such as beads, on a solid metal substrate to create a porous surface. Typical of such coatings are those disclosed in US-3855638, US-4206516, US-4156943, and US-4612160. US-5368881 and US-5658333 show use of non-spherical powder to produce a roughened surface for prosthesis.

Such porous irregular surfaces may be roughened, for example by grit blasting; however, grit blasting can cause significant changes to the surface topography by damaging the metal elements (e.g., beads) on the surface layer of the substrate. Other mechanical roughening techniques, such as scratching or burr grinding, have also been used. These techniques can also present drawbacks, including distortion of the substrate, removal of excess material, inability or difficulty to roughen certain surfaces, and inconsistent surface roughening. Porous surfaces may also be roughened by etching, as disclosed for example, in US-A-2006/0289388 and US-A-2004/0167633.

There are also implants that comprise fully porous or open-celled metallic structures. For example, there are tantalum metal open-celled metallic structures ("Trabecular Metal") that do not require a metal substrate. Other open-celled metallic structures are available in other metals as well; for example, US-A-2006/0002810 and US-6849230, US-5104410 and US-3852045 disclose porous metal implants made from powder metal. US-A-2008/0199720 also discloses porous metal implants made from powder metal. Whether made from sintered wire mesh, sintered metal powder or some other material, such implants may be porous throughout substantially the entire structure (that is, open celled throughout at least one cross-section of the implant) and are referred to herein as "open-celled metal implants". When these implants contain titanium (that is, when the implants are made from titanium or a titanium alloy), such open-celled metal implants are referred to herein as "open-celled titanium implants". When made from powder metal, such open-celled metal implants are referred to herein as "open-celled sintered powder metal implants". When made from a powder metal that contains titanium (that is, when the powder metal includes titanium or a titanium alloy), such open-celled sintered powder metal implants are referred to herein as "open-celled sintered titanium powder implants".

Open-celled metal implants (including open-celled sintered powder metal implants) are advantageous in providing a high volume porosity capable of supporting tissue ingrowth. While it is anticipated that bone ingrowth and initial stability of the open-celled metal implants could be optimized by increasing the surface roughness of the open-celled metal implants, post-sintering machining is not feasible because the process could close the pores and smear the surface of the implant. Grit blasting is not suitable for use on such open-celled metal implants because the glass beads may become embedded in the pores. Etching the open-celled metal implants could weaken the already highly-porous implant.

The present invention addresses the need to optimize bone ingrowth and initial stability of open-celled metal implants without closing pores and without embedded grit while also maintaining adequate mechanical properties.

One aspect of the invention relates to etching processes to roughen the surface of an open-celled metal implant. Articles comprising such open-celled metal implants include orthopaedic implants and dental implants. Another aspect of the invention relates to the roughened open-celled metal implants produced by the etching process. These implants may be orthopaedic implants and dental implants.

In one aspect, the invention provides a process for etching a porous titanium foam or porous titanium alloy foam comprising:
drying said foam;
contacting said foam with an aqueous acid solution comprising about 0.5 to about 5 volume percent HF and about 5 to about 20 volume percent HNO₃ for a time sufficient to achieve a desired surface roughness;
removing said foam from the acid solution and contacting said foam with water to remove residual acid, and
removing any dark colour appearing on the surface of said foam by heating said foam at 800 to 1000°C for not less than 0.5 hour.

Preferably, the aqueous acid solution comprises about 1 to about 3 percent of HF by volume.

Preferably, the aqueous acid solution comprises about 7 to about 13 percent of HNO₃ by volume.

Preferably, the aqueous acid solution comprises about 1 to about 3 percent of HF by volume and about 7 to about 13 percent of HNO₃ by volume.

Preferably, the foam has a porosity of about 60 to about 75 percent prior to the contacting step.

Preferably, the titanium alloy is Ti-6A1-4V.

Preferably, the foam is contacted with said aqueous acid solution for a time that is not more than about 30 minutes, preferably from about 2 to about 15 minutes.

Preferably, the process includes the step of removing at least a portion of dust or grease on said foam prior to said contacting step.

Preferably, the process includes the step of cleaning the foam with detergent and alcohol prior to contacting the foam with the aqueous acid solution.

Preferably, the process includes the step of drying the foam after cleaning and before contacting the foam with the aqueous acid solution.

Preferably, the step of contacting the foam with the aqueous acid solution comprises immersing the foam in the aqueous acid solution under conditions wherein penetration of the aqueous acid solution into the foam is limited.

Preferably, the process includes the step of contacting said foam with an aqueous rinse solution after contacting said foam with the acid solution.

Preferably, the process includes the step of cleaning the foam with a water jet.

Preferably, the heating step comprises heating said foam at a temperature that is at least 800°C.

In another aspect, the invention provides process for etching an open-celled metal medical implant comprising:
cleaning the implant;
drying the cleaned implant in an oven;
immersing the dry implant in an aqueous acid etchant bath;
cleaning the etched implant to remove acid;
spraying the etched implant with a water jet to remove weak connections on the surface of the etched implant; and
heating the etched implant to remove undesirable residue.

Preferably, the step of cleaning the implant comprises cleaning the implant with a detergent and placing the implant in an alcohol bath.

Preferably, the step of immersing the dry implant comprises placing the implant in the etchant bath under conditions wherein air is trapped within the implant to limit migration of the etchant beyond the surface of the implant.

Preferably, the water jet provides a pressure of about 13.8 MPa to 27.6 MPa (2000 to 4000 psi).

Preferably, the step of heating the etched implant comprises heating the etched implant in a vacuum furnace at a temperature of at least 800°C.

Preferably, the open-celled metal medical implant comprises an open-celled titanium medical implant, more preferably an open-celled sintered titanium powder medical implant, especially an open-celled sintered metal powder orthopaedic implant.

Preferably, the aqueous etchant bath comprises 0.5 to 5% by volume hydrofluoric acid and 5 to 20% by volume nitric acid, more preferably, the 1 to 3% by volume hydrofluoric acid and 10% by volume nitric acid.

Preferably, the implant remains in the aqueous etchant bath for 2 to 30 minutes at ambient temperature.

Preferably, the implant remains in the aqueous etchant bath for 20 minutes or less.

In a further aspect, the invention provides an open-celled metal medical implant comprising an outer surface and a body, in which the outer surface of the implant has an average porosity greater than the bulk porosity of the implant.

Preferably, the outer surface comprises a layer less than 1 mm thick.

Preferably, the implant comprises an open-celled metal orthopaedic implant, more preferably an open-celled titanium/Ti alloy orthopaedic implant.

Preferably, the implant comprises an open-celled sintered titanium powder orthopaedic implant.

Preferably, the implant comprises an open-celled sintered metal powder orthopaedic implant.

Preferably, the implant has a coefficient of static friction of at least 0.8 as measured by ASTM D4518-91.

Preferably, the bulk porosity of the implant is at least 60% and the average porosity of the outer surface is at least 5% greater than the bulk porosity.

Preferably, the implant has a 0.2% compression yield strength of more than 50 MPa.

In another aspect, the invention provides a porous titanium foam having a coefficient of static friction greater than 0.4 as measured by ASTM D4518-91.

Preferably, the implant has a coefficient of static friction of at least 0.8 as measured by ASTM D4518-91.

Preferably, the foam defines an open-celled sintered titanium powder implant having an outer surface and a body, and the outer surface of the implant has an average porosity greater than the bulk porosity of the implant.

Preferably, the bulk porosity of the implant is at least 60% and the average porosity of the outer surface is at least 5% greater than the bulk porosity.

Preferably, the foam has a 0.2% compression yield strength of more than 50 MPa.

Preferably, the outer surface comprises a layer less than 1 mm thick.

Preferably, the foam has a coefficient of static friction of at least 1 as measured by ASTM D4518-91.

Preferably, the foam has a 0.2% compression yield strength of more than 50 MPa; and the bulk porosity of the implant is at least 60% and the average porosity of the outer surface is at least 5% greater than the bulk porosity.

In one aspect, the invention provides a process for etching an open-celled metal implant comprising contacting the dry implant with an aqueous acid solution comprising about 0.5 to about 5 volume percent HF and about 5 to about 16 volume percent HNO₃ for a time sufficient to achieve a desired surface roughness. In some embodiments, the aqueous acid solution comprises about 1 to about 3 percent by volume of HF. In certain embodiments, the aqueous acid solution comprises about 7 to about 13 percent by volume of HNO₃.

Some embodiments concern processes for etching a porous titanium foam or porous titanium alloy foam comprising:
cleaning the foam;
drying the foam;
contacting the foam with an aqueous acid solution comprising about 0.5 to about 5 volume percent HF and about 5 to about 20 volume percent HNO₃ for a time sufficient to achieve a desired surface roughness;
removing the foam from the acid solution and contacting the foam with water to remove residual acid, and
optionally, removing any dark color appearing on the surface of the foam by heating the foam at 800 to 1000°C for at least 0.5 hour and preferably 1 to 2 hours.

The open-celled metal implants etched with the solutions described herein may be open-celled titanium implants containing titanium and/or titanium alloys. Examples of suitable metals include pure titanium and titanium alloys such as Cp-Ti (commercially pure titanium) and Ti6A14V (titanium alloyed with 6% aluminum and 4% vanadium; as specified in ASTM-F 1580-01). Suitable titanium or titanium alloy foams include those having a porosity of about 60 to about 75 percent prior to contacting the foam with the acid solution. The open-celled titanium implants etched by the process of the invention may comprise open-celled sintered titanium powder implants.

For open-celled titanium implants, the process preferably includes a step to remove dark stains on the etched surface which may be the byproducts induced by etching. This step comprises heating the etched open-celled titanium implant at a high temperature (for example, 800 to 1000°C) in a vacuum furnace for a period of time (for example, 1 hour). With this step, a roughened open-celled titanium implant can be produced that is substantially free from etched byproducts .

In some embodiments, the foam is contacted with the aqueous acid solution for a time that is no more than about 30 minutes. Some methods involve contacting the foam with the aqueous acid solution for a time of about 1 to about 10 minutes or for a time of about 2 to about 5 minutes.

It can be advantageous to remove at least a portion of dust or grease on the foam prior to contacting the foam with the acid solution. Some embodiments utilize an aqueous rinse solution to remove residual acid after contacting the foam with the acid solution.

Some embodiments remove at least a portion of the water introduced by the rinse solution. One way of removing the water is contacting the foam with a gaseous stream for a time and under conditions effective to remove at least a portion of the water from the foam. Another way of removing water from the rinsed foam is by heating the foam to a temperature above 25 °C.

The process of the present invention also allows for controlling the depth of roughening of the open-celled metal implants. By swiftly fully immersing a dry implant in the etching solution, some air will remain trapped within the cells in the interior of the implant, and the combination of air pressure and surface tension will limit or slow the penetration of the etching solution, and thereby increasing the roughness to on the surface (and to a limited depth beneath the surface) of the implant while limiting any weakening of the interior structure of the implant. Using this process, a roughened open-celled metal implant can be produced wherein the bulk porosity is 60to 75% while the porosity to about 1 mm from the surface is about 5 to 10% higher. Moreover, in such an implant, pore edges at the surface and to a depth of about 1 mm will be sharpened while pore edges at the core of the implant will remain unsharpened.

In another aspect, the present invention provides open-celled titanium implants (including open-celled sintered titanium powder implants) that have roughened surfaces and that are substantially free from etched byproducts residue. In addition, the present invention provides open-celled metal implants (including open-celled sintered power metal implants) that have roughened surfaces and that have surfaces that are more porous than the bulk porosity of the implant.

In a further aspect, the invention provides a process for etching an open-celled metal medical implant comprising: cleaning the implant; drying the cleaned implant in an oven; immersing the dry implant in an aqueous acid etchant bath; cleaning the etched implant to remove acid; spraying the etched implant with a water jet to remove weak connections on the surface of the etched implant; and heating the etched implant to remove undesirable residue.

In another aspect the invention provides open-celled metal medical implants having an outer surface and a body, wherein the outer surface of the implant has an average porosity greater than the bulk porosity of the implant. Certain of these implants have an outer surface layer that is less than 1 mm thick. Some porous titanium foams have a coefficient of static friction greater than 0.4 or 0.8 as measured by ASTM D4518-91. Certain implants have a 0.2% compression yield strength of more than 50 MPa.

In a further aspect, the invention provides porous titanium foams having a coefficient of static friction greater than 0.4 as measured by ASTM D4518-91. Some foams have a coefficient of static friction greater than 0.8 or 1.0 as measured by ASTM D4518-91. Certain foams define an open-celled sintered titanium powder implant having an outer surface and a body, and wherein the outer surface of the implant has an average porosity greater than the bulk porosity of the implant. In some embodiments, the bulk porosity of the implant is at least 60% and the average porosity of the outer surface is at least 5% greater than the bulk porosity. Some foams have a 0.2% compression yield strength of more than 50 MPa.

Terms used in this specification have the following meanings:
"Open-celled metal medical implant(s)" is an open-celled metal implant sized and shaped for use as either an orthopaedic implant or a dental implant.
"Open-celled metal orthopaedic implant(s)" is an open-celled metal medical implant sized and shaped for use in joint replacement, spine surgery or trauma and includes stems sized and shaped to be received within the intramedullary canal of a bone, such as the femur, tibia or humerus, as well as tibial plates, acetabular cups, spinal implants, intramedullary nails, bone plates and glenoid components, for example. "Open-celled metal orthopaedic implant(s)" is intended to include open-celled metal medical implant(s) and open-cell sintered powder metal medical implant(s).
"Open-celled titanium medical implant(s)" is an open-celled titanium implant sized and shaped for use as either an orthopaedic implant or a dental implant.
"Open-celled titanium orthopaedic implant(s)" is an open-celled titanium medical implant sized and shaped for use in joint replacement, spine surgery or trauma and includes stems sized and shaped to be received within the intramedullary canal of a bone, such as the femur, tibia or humerus as well, as tibial plates, acetabular cups, spinal implants, intramedullary nails, bone plates and glenoid components, for example. "Open-celled titanium medical implant(s)" is intended to include open-celled sintered titanium powder medical implant(s).
"Open-celled sintered metal powder medical implant(s)" is an open-celled medical implant made by a powder metallurgy process and sized and shaped for use as either an orthopaedic implant or a dental implant.
"Open-celled sintered metal powder orthopaedic implant(s)" is an open-celled sintered metal powder medical implant made by powder metallurgy process and sized and shaped for use in joint replacement, spine surgery or trauma and includes stems sized and shaped to be received within the intramedullary canal of a bone, such as the femur, tibia or humerus, as well as tibial plates, acetabular cups, spinal implants, intramedullary nails, bone plates and glenoid components, for example. "Open-celled sintered metal powder orthopaedic implant(s)" is intended to include open-celled sintered titanium powder orthopaedic implant(s).
"Open-celled sintered titanium powder medical implant(s)" is an open-celled sintered metal powder implant made by a powder metallurgy process from a titanium powder and sized and shaped for use as either an orthopaedic implant or a dental implant.
"Open-celled sintered titanium powder orthopaedic implant(s)" is an open-celled sintered metal powder medical implant made by a powder metallurgy process and sized and shaped for use in joint replacement, spine surgery or trauma and includes stems sized and shaped to be received within the intramedullary canal of a bone, such as the femur, tibia or humerus, as well as tibial plates, acetabular cups, spinal implants, intramedullary nails, bone plates and glenoid components, for example.
"Titanium" is either pure titanium or a titanium alloy and includes pure titanium powder as well as a metal powder that comprises a titanium alloy.

Fixation is an important requirement for orthopaedic implants and dental implants, whether open-celled or having a fully dense portion (for example, a solid metal substrate). For cementless fixation of medical implants, surface roughness can increase the frictional force between the medical implant and the bone, thereby providing initial stability of the medical implants. The rough or porous surface also allows a medical implant to mate with the bone and invite bone ingrowth such that, after a period of time, the medical implant becomes integrated into the bone structure. In addition, the medical implant should have sufficient structural strength to bear the anticipated loads over years of use and to resist fretting when implanted. The present invention addresses the need for both surface roughness and structural strength in open-celled medical implants, and, more specifically, addresses these needs in open-celled sintered titanium powder orthopaedic implants.

Open-celled sintered titanium powder orthopaedic implants and processes for making them are disclosed in US-A-2008/0199720. US-A-2006/0002810 A1 and US-6849230, US-5104410 and US-3852045 disclose other open-celled sintered metal powder medical implants. The open-celled sintered metal powder orthopaedic implants disclosed in those applications and patents are porous, and have an open cell structure with pores serving as tissue receptors for bone ingrowth. Such open-celled sintered metal powder orthopaedic implants may have 60-75% porosity. And while the clinical results of using such implants is expected to be good, the inventors believe that the initial stability of such implants and bone ingrowth could be improved by increasing the surface roughness of these implants. Conventional machining or glass bead blasting would not be optimal for roughening the outer surfaces of such open-celled sintered powder metal medical implants due to the highly porous nature of the open cell structure. The inventors have discovered an etch process that roughens the implant surface by sharpening the edges of the pores, making the implant particularly suitable for cementless fixation, while maintaining sufficient structural strength to resist fretting during implantation and to perform in its intended application. Moreover, the complete etching process includes a step for removing any undesirable residue so that the etched implants are substantially free from undesirable residue (such as titanates).

The process of the present invention includes the following steps. First, an open-celled metal medical implant is provided. The open-celled metal medical implant is cleaned, dried and immersed in an etchant. The implant is removed from the etchant and cleaned, dried and then heated in a vacuum furnace to remove undesirable side products.

The initial cleaning step may be performed as follows. The open-celled metal medical implant may be cleaned with a detergent, rinsed in deionized water and then immersed in an ultrasonic bath in 100% alcohol. Such treatment is useful in removing grease and dust prior to etching. An example of suitable detergents is Liquid-Nox. An example of suitable alcohols is 100% Reagent Alcohol.

A suitable etchant for open-celled titanium medical implants is an aqueous solution containing low concentrations of hydrofluoric acid (0.5-5% HF) and nitric acid (5-20% HNO₃). The concentration of hydrofluoric acid can be at least 1%, preferably at least 2%. The concentration of hydrofluoric acid can be not more than 4%, preferably not more than 3%. The concentration of nitric acid can be at least 8%, preferably at least 9%. The concentration of nitric acid can be not more than 14%, preferably not more than 12%. The percentages of the acids in the etchant solution is by volume. The remainder of the solution is deionized water. After drying, air is present within the pores of the implant and the surface tension of the implant is greater than prior to drying. Swift immersion of the dried implant in the etchant bath traps some air within the interior of the body of the implant. Together with the surface tension of the dried implant, the air pressure within the body of the implant limits infiltration of the etchant, thereby limiting the degree of etching that will occur in the deep pores in the interior of the body of the implant while allowing the degree of desirable etching of the pores on the surface of the implant. For an open-celled titanium medical implant, the etching time may range, for example, from 2 to 30 minutes at ambient temperature (about 25 °C). Variables such as acid concentration in the etchant, time in the etchant solution, and temperature at which etching is performed may be varied depending on the degree of etching desired. It should be understood that quantities identified for these variables herein are provided as examples only to produce a particular degree of etching in the identified medical implant; the invention is not limited to any particular acid concentration, etching time or etching temperature unless specified in the claims. For an open-celled sintered powder metal medical implant with 60 to 75% porosity, the etching time is less than about 30 minutes, typically around 2 to 15 minutes at ambient temperature, and the volume ratio of the etchant solution to implant is larger than 5.

After the implant has been etched for the desired time, it is removed from the etchant solution and cleaned. This cleaning step may comprise placing the etched medical implant in an ultrasonic bath with deionized water for a time sufficient to remove any residual etchant from the implant. For example, the medical implant may be immersed in the bath for 3 hours; the water in the bath may be changed periodically during the cleaning process (for example, once every hour). After the implant has been immersed for the desired time in the bath, it may be removed and rinsed with deionized water.

To remove any weak connections in the structure of the implant, the implant may be cleaned with a water jet. For example, a water jet can be used with a pressure of at least 13.8 MPa (2000 psi), preferably at least 17.2 MPa (2500 psi), for example about 20.7 MPa (3000 psi). A water jet can be used with a pressure of not more than 27.6 MPa (4000 psi), preferably not more than 24.1 MPa (3500 psi). After such a cleaning step, any debris should be removed so that debris does not fall off in the patient's body when the implant is subject to friction during implantation.

The cleaned implant is then dried and heated to remove undesirable byproducts that have a dark (brown and black) colour shown on the implant surface. For an open-celled titanium medical implant, the undesirable byproducts can be removed by heating the etched, cleaned and dried implant in a vacuum furnace at about 800 to 1000°C for more than 1 hour, for example. The time and temperature may be varied according to the type of metal and type of residue that is to be removed.

Use of the above-described etchant solution has been found to roughen the outer surface of an open-celled titanium orthopaedic implant in all directions uniformly on a micron scale. This result can be seen by visually inspecting the etched parts under optical microscope. Furthermore, the instant etch process can open up more pores with initial small openings and connections compared to conventional techniques. These openings and connections improve the overall permeability of the implants. Although the particular etchant is useful for roughening the surfaces of open-celled titanium implants, other aspects of the method of the present invention may be useful in roughening the surfaces of other open-celled metal implants with other etchants.

Embodiments of the invention are described below by way of example with reference to the accompanying drawings, in which:
Figure 1 presents a micrograph of a Ti-foam coupon sample prepared by cutting it from an open-celled titanium rod made by powder metallurgy process with a porosity of 65%, dissolving the pore forming agent (PFA) and sintering the coupon at 1371 °C (2500°F) for 6 hours.
Figure 2 presents a micrograph of a Ti-foam coupon sample after etching the coupon sample for 20 minutes using an aqueous etchant containing 1% HF and 10% HNO₃.
Figure 3 presents a micrograph of a Ti-foam coupon sample after etching the coupon sample for 10 minutes using an aqueous etchant containing 2% HF and 10% HNO₃.
Figure 4 presents a micrograph of a Ti-foam coupon sample after etching the coupon sample for 3 minutes using an aqueous etchant containing 3% HF and 10% HNO_{3.}
Figure 5 presents a photograph of a cross-section of a Ti-foam coupon sample after etching the coupon sample for 5.5 minutes using an aqueous etchant containing 2% HF and 10% HNO₃.
Figure 6 presents a micrograph of a portion of an open-celled titanium acetabular cup made by powder metallurgy process after bead blasting in the green state.
Figure 7 presents a micrograph of a portion of an open-celled titanium acetabular cup made by powder metallurgy process after bead blasting in the green state followed by sintering and etching.
Figure 8 presents a micrograph of a portion of an open-celled titanium acetabular cup made by powder metallurgy process after etching.
Figure 9 presents a perspective view of an open-celled titanium orthopaedic implant that has been etched in accordance with the present invention.
Figure 10 shows that dark colour produced as an etching byproduct can be removed by post-etching heat treatment.

### EXAMPLES

### Example 1

Open-celled sintered titanium powder samples in the form of femoral augments were prepared following the procedure described in US-A-2008/0199720. Commercially pure titanium powder (Phelly Materials, Inc., Bergenfield NJ, USA) particle size less than 45 µm and NaCl (Fisher Scientific International, Hampton, NH, USA) particle size 250 to 425 µm as a pore-forming agent (PFA), were mixed in a ratio of approximately 25:75 Ti:PFA by volume. Reverse osmosis (RO) water was added in an amount corresponding to about 700 µL per 100 cm³ of Ti:PFA mixture. The mixture was added to a mould and compressed into a green body at a compaction pressure of 310 MPa (45 ksi). The green body was placed in a water bath until the NaCl dissolves. The resulting metal skeleton was dried at 65°C for 4 hours, and then sintered at 1371 °C for 6 hours. Four samples were prepared. One of these samples was retained as a control and the other three samples were treated as described in Example 2 below.

### Example 2

Three of the samples of Example 1 were cleaned with a detergent (Liguid-Nox, Alconox, Inc.), rinsed in RO water and immersed in an ultrasonic bath in 100% alcohol (100% Reagent Alcohol, Fisher Scientific)) for one hour. The samples were removed from the bath and dried in an oven at about 65 °C for 4 hours. Three batches of etchant were prepared as set out in the following table, and samples 2 to 4 were etched under the conditions set forth in the following table and below:

| Sample | HF concentration (by volume) | HNO₃ concentration (by volume) | Temperature | Immersion time |
|---|---|---|---|---|
| 1 | None | None | Not applicable | None |
| 2 | 1% | 10% | Ambient | 20 minutes |
| 3 | 2% | 10% | Ambient | 10 minutes |
| 4 | 3% | 10% | Ambient | 3 minutes |

The hydrofluoric acid used in preparing each of the etchant baths was a 47 to 52% concentration aqueous solution purchased from Laboratory Chemicals of J T Baker. The nitric acid used in preparing each of the etchant baths was a 50 to 70% concentration aqueous solution purchased from Fisher Chemical of Fisher Scientific.

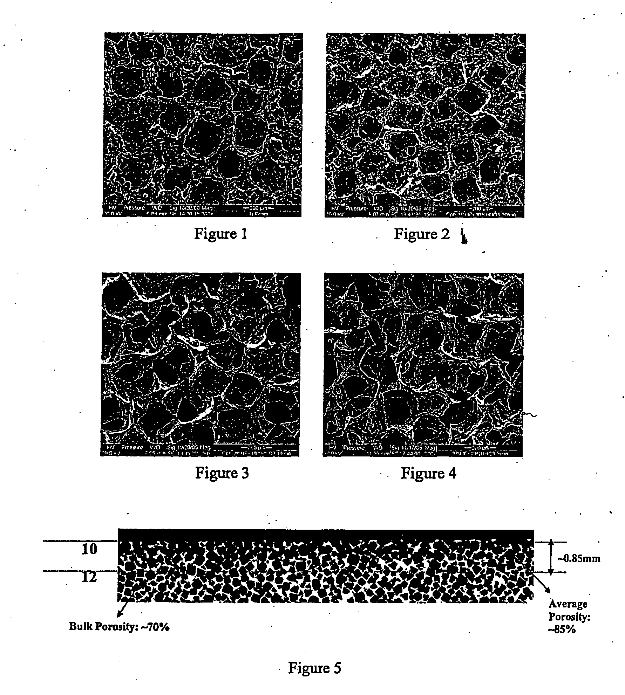

Samples 2 to 4 were quickly totally immersed in the etchant and maintained in the aqueous etchant bath at ambient temperature for the times set forth above. After etching for these times, the samples were removed from the etchant bath and placed in an ultrasonic bath of RO water for 3 hours at ambient temperature; the water was changed every one hour. Samples 2to 4 were then removed from the ultrasonic bath and cleaned with a water jet at 20.68 MPa (3000 psi) for about 5 minutes. Samples 2 to 4 were then dried and placed in a vacuum furnace at 1000°C for 1.5 hours and then removed from the furnace. Scanning electron microscope (SEM) images of the outer surfaces of Samples 1 to 4 are provided in FIGS. 1 to 4, with FIG. 1 being Sample 1, FIG. 2 being Sample 2, FIG. 3 being Sample 3 and FIG. 4 being Sample 4.

As can be seen from a comparison of FIG. 1 with FIGS. 2 to 4, the surfaces defining the pores of an open-celled sintered titanium powder implant after etching have become much sharper and therefore much rougher than Sample 1, which was not etched. In addition, the etching process has further opened the pores on the surface and has enhanced the interconnectivity of the pores.

### Example 3

An open-celled sintered titanium powder sample was prepared as in Example 1, except its green porosity was 80% instead of 75%. This sample was treated similarly to Sample 2 above (2% HF aqueous etchant solution) but etched for a period of 5.5 minutes. This sample is referred to as Sample 5. A cross-section of Sample 5 was prepared and observed through an optical microscope. FIG. 5 is an image of this cross-section. As can be seen in FIG. 5, the outer layer 10 (the outer surface of Sample 5) of Sample 5 has greater porosity than the interior 12 of the sample. The depth of the outer layer 10 in Sample 5 was 0.85 mm. The porosity of the outer layer 10 and the interior 12 were both measured by imaging analysis, using software sold under the trade mark "Image-ProPlus". The bulk porosity of the entire sample was also measured using the same method prior to the etching by cutting three cross section specimens at different locations and taking 10 images from each section specimen to obtain an average porosity (about 70%) for the bulk sample. The porosity of the outer layer 10 was about 85% while the bulk porosity was about 70%, illustrating that the etching process had been effective at the surface of Sample 5 but had not penetrated significantly below the surface.

The degree and the depth of the roughness created by the etching step can be adjusted by changing the etching conditions. For example, increasing the etching time can increase the degree of etching on the surface as well as the depth of the etching into the interior of the body of the sample.

### Example 4

Two open-celled sintered titanium powder samples were prepared as in Example 1 and treated similarly to Sample 4 above (3% HF aqueous etchant solution) but etched for periods of 2 minutes and 3 minutes. These samples are referred to as Samples 6 and 7 herein. The 0.2% compression yield strength of Samples 6 and 7 was measured by performing the standard compression test of ASTM E9-89a. The 0.2% compression yield strength of an unetched open-celled sintered titanium powder sample (Sample 8) was also measured. The 0.2% compression yield strength of a sample of an open-celled tantalum metal orthopaedic implant material, commercially available from Zimmer Inc of Warsaw, Indiana under the trade mark Trabecular Metal (Sample 9), was also measured. The results are provided in the following table:

| Specimen | Etching Conditions | 0.2% Compression Yield Strength |
|---|---|---|
| 6: Ti foam | 3% HF solution | 75.3 MPa |
| | 10 % HNO₃ | |
| | 2 minute etch | |
| 7: Ti foam | 3% HF solution | 59.5 MPa |
| | 10 % HNO₃ | |
| | 3 minute etch | |
| 8: Ti foam | None | 77 MPa |
| 9: Trabecular Metal | None | 49 MPa |

These results show that the process of the present invention does not cause the etched parts to lose much mechanical strength. The 0.2% compression yield strength of the sample etched for 2 minutes was about 98% of that for the unetched sample made from titanium powder and the 0.2% compression yield strength of the sample etched for 3 minutes was about 77% of that for the unetched sample made from titanium powder; both etched samples had a 0.2% compression yield strength greater than that of commercially available Trabecular Metal (unetched).

### Example 5

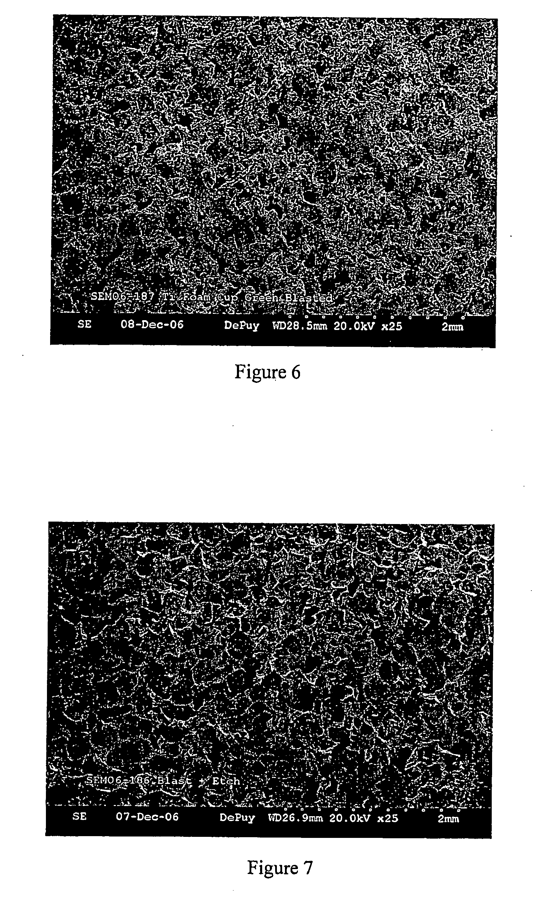

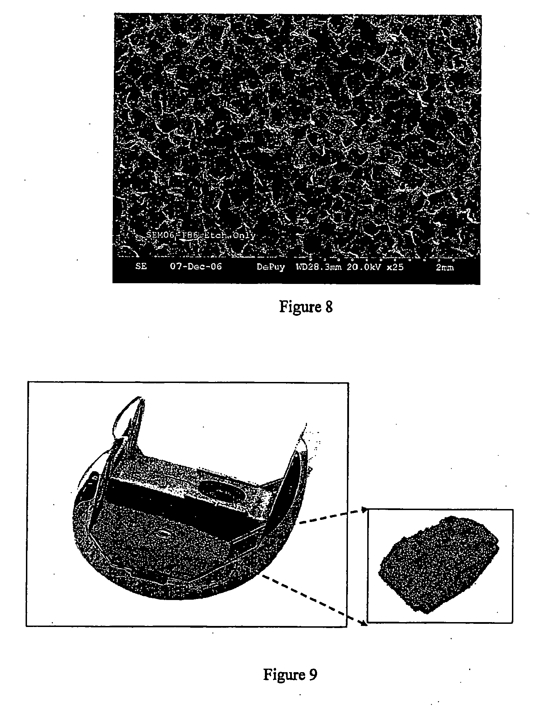

Three samples of open-celled sintered titanium powder orthopaedic implants (acetabular cups) were prepared as in Example 3. However, one sample (Sample 9) was salt blasted while in the green state (prior to sintering). A second sample (Sample 10) was also salt blasted while in the green state, sintered, and then etched as in Example 2 above. A third sample (Sample 11) was etched as in Example 2 above without any salt blasting. SEM images were taken from each sample. FIG. 6 is an SEM image of Sample 9; FIG. 7 is an SEM image of Sample 10; and FIG. 8 is an SEM image of Sample 11. As can be seen from these SEM images, etching produces sharper edges on the metal defining the pores than does salt blasting.

In addition, mechanical techniques such as blasting are "line of sight" processes, while the etching process of the present invention acts on all sides of the pores; the surfaces of the etch-treated open-celled sintered titanium powder products are uniformly rougher on all the sides, not just on one side. In addition, the degree of the implant surface roughness can be easily controlled by altering the acid concentrations in the etchant and the length of time that the implant is contacted with the etchant.

### Example 6

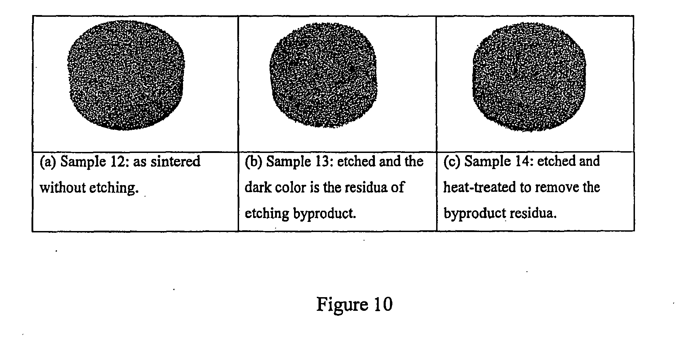

Samples 12 to 14 were prepared as in Example 3, with the exception that the PFA used to make these samples were in 300 to 500 µm range. Sample 12 was not etched, sample 13 was etched but not heat-treated, and sample 14 was heated in the vacuum furnace at 1000°C for one hour after etching and water jet cleaned. As shown in the Figure 10, the dark colour revealed from sample 12 was induced by etching as the residua of byproduct and it can be removed by post-etching heat treatment. XPS analysis carried out on these samples indicates the byproduct may be some sort of titanate but the exact chemical compound is difficult to determine and confirm. However, after the post-etching heat treatment, it cannot be detected by XPS.

### Example 7

Samples 15 to 18 were prepared as in Example 6 and etched in different conditions as specified in the table below with the exception that Sample 17 was not etched The static friction coefficient of each sample was measured following the ASTM standard D4518-91.

The table below summarizes the coefficients of static friction measured for the samples identified:

| Specimen | Etching Conditions | Coefficient of Static Friction |
|---|---|---|
| 15a-15c | 2% HF solution | 1.07±0.09 |
| | 10 % HNO₃ | |
| | 10 minute etch | |
| 16a-16c | 3% HF solution | 1.01±0.04 |
| | 10 % HNO₃ | |
| | 3 minute etch | |
| 17a-17c | None | 0.44±0.03 |
| 18a-18c | None (Trabecular Metal) | 0.81±0.16 |

As the above results show, the etched specimens all had significantly higher coefficients of static friction compared to the unetched sintered titanium powder and Trabecular Metal specimens. With these higher coefficients of friction, it is anticipated that etched open-celled metal medical implants will have greater initial stability when implanted to bear against native bone and will be more conducive to bone ingrowth compared to unetched open-celled metal medical implants.

Although the above open-celled sintered titanium powder specimens were made following the procedure disclosed in US-A-2008/0199720, the etching process should produce equivalent results with open-celled metal medical implants made following other procedures.

Although the processes of the invention make it convenient to roughen the implant surface uniformly on all sides by emerging the whole implant in the etchant solution, one can also use the process to roughen one or more selected portions of an implant by contacting only that side or sides with the solution. For example, for a tibial tray, the proximal bearing surface need not be immersed in the etchant bath.

In addition, although the process described above has been applied to open-celled orthopaedic implants, the present invention may also be applied to medical implants comprising a solid metal substrate with a porous foam coating on the substrate, and more particularly, to the porous foam portion of the implant. Thus, the aqueous acid etchant solution described above may be used with titanium foams defining open-celled medical implants as well as with titanium foam coatings on solid substrates.

There are several potential advantages of the etch process of this patent application. The process can often result in uniform rough surface with sharp pore edges; such roughness is desired for cementless fixation implants. Also, etching often only occurs in the surface region of Ti foam, so it does not significantly reduce the Ti foam mechanical strength. In addition, the degree and depth of surface roughness typically is adjustable by changing the etching condition. Finally, etching generally further opens up the pores in the surface region of Ti foams and enhances the interconnectivity.

## Claims

1. A process for etching a porous titanium foam or porous titanium alloy foam comprising:
drying said foam;
contacting said foam with an aqueous acid solution comprising about 0.5 to about 5 volume percent HF and about 5 to about 20 volume percent HNO₃ for a time sufficient to achieve a desired surface roughness;
removing said foam from the acid solution and contacting said foam with water to remove residual acid, and
removing any dark color appearing on the surface of said foam by heating said foam at 800 to 1000°C for not less than 0.5 hour.

2. The process of claim 1, wherein the aqueous acid solution comprises about 1 to about 3 percent of HF by volume and about 7 to about 13 percent of HNO₃ by volume.

3. The process of claim 1, wherein said foam has a porosity of about 60 to about 75 percent prior to said contacting step.

4. The process of claim 1, wherein the foam is contacted with said aqueous acid solution for a time of about 2 to about 15 minutes.

5. The process of claim 1, further comprising removing at least a portion of dust or grease on said foam prior to said contacting step.

6. The process of claim 1 further comprising cleaning the foam with detergent and alcohol prior to contacting the foam with the aqueous acid solution.

7. The process of claim 6 further comprising drying the foam after cleaning and before contacting the foam with the aqueous acid solution.

8. A process for etching an open-celled metal medical implant comprising:
cleaning the implant;
drying the cleaned implant in an oven;
immersing the dry implant in an aqueous acid etchant bath;
cleaning the etched implant to remove acid;
spraying the etched implant with a water jet to remove weak connections on the surface of the etched implant; and
heating the etched implant to remove undesirable residue.

9. An open-celled metal medical implant comprising:
an outer surface and a body, wherein the outer surface of the implant has an average porosity greater than the bulk porosity of the implant.

10. The open-celled metal medical implant of claim 9 wherein the outer surface comprises a layer less than 1 mm thick.

11. A porous titanium foam having a coefficient of static friction greater than 0.4 as measured by ASTM D4518-91.

12. The porous titanium foam of claim 11, in which the foam defines an open-celled sintered titanium powder implant having an outer surface and a body, and wherein the outer surface of the implant has an average porosity greater than the bulk porosity of the implant.
